(19) Europäisches Patentamt
European Patent Office
Office européen des brevets

(11) Veröffentlichungsnummer: **0 471 259 B1**

(12) # EUROPÄISCHE PATENTSCHRIFT

(45) Veröffentlichungstag der Patentschrift: **17.05.95**

(51) Int. Cl.[6]: **C07D 213/53**, C07D 213/55, C07D 213/71, A61K 31/44

(21) Anmeldenummer: **91113089.6**

(22) Anmeldetag: **03.08.91**

(54) **Phenylsulfonamid-substituierte Pyridinalken- und -aminooxyalkancarbonsäure-derivate.**

(30) Priorität: **16.08.90 DE 4025818**

(43) Veröffentlichungstag der Anmeldung:
**19.02.92 Patentblatt 92/08**

(45) Bekanntmachung des Hinweises auf die Patenterteilung:
**17.05.95 Patentblatt 95/20**

(84) Benannte Vertragsstaaten:
**AT BE CH DE DK ES FR GB GR IT LI LU NL SE**

(56) Entgegenhaltungen:
**EP-A- 0 397 044**
**US-A- 4 518 602**
**US-A- 4 874 775**

(73) Patentinhaber: **BAYER AG**
**D-51368 Leverkusen (DE)**

(72) Erfinder: **Niewöhner, Ulrich, Dr.**
**Gartenstrasse 3**
**W-5632 Wermelskirchen 2 (DE)**
Erfinder: **Müller, Ulrich E., Dr.**
**Claudiusweg 5**
**W-5600 Wuppertal 1 (DE)**
Erfinder: **Perzborn, Elisabeth, Dr.**
**Am Tescher Busch 13**
**W-5600 Wuppertal 2 (DE)**
Erfinder: **Bischoff, Erwin, Dr.**
**Pahlkestrasse 73**
**W-5600 Wuppertal 1 (DE)**
Erfinder: **Dellweg, Hans-Georg, Dr.**
**In den Birken 46**
**W-5600 Wuppertal 1 (DE)**

**EP 0 471 259 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

**Beschreibung**

Die Erfindung betrifft Phenylsulfonamid-substituierte Pyridinalken- und -aminooxyalkancarbonsäurederivate, Verfahren zu ihrer Herstellung und ihre Verwendung in Arzneimitteln.

Es ist bereits bekannt, daß Pyridinalkencarbonsäuren und Pyridinmethylenaminooxyalkancarbonsäuren eine thrombozytenaggregationsinhibitorischeWirkung besitzen [vgl. EP 221 601 und EP 135 316].

Die Erfindung betrifft Phenylsulfonamid-substituierte Pyridinalken- und -aminooxyalkancarbonsäurederivate der allgemeinen Formel (I),

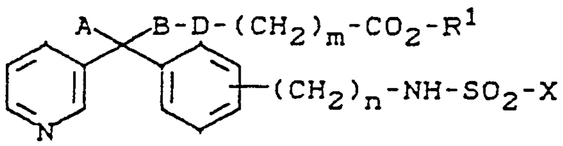

(I)

in welcher

| | | |
|---|---|---|
| A | | für Wasserstoff steht, |
| B | | für die $-CH_2$-Gruppe steht, oder |
| A und B | | gemeinsam für einen Rest der Formel =CH- oder =N- stehen, |
| D | | für die $-CH_2$-Gruppe steht oder im Fall, daß B mit A den Rest der Formel =N- bedeutet, für Sauerstoff steht, |
| m | | - für eine Zahl 1, 2, 3, 4, 5, 6, 7 oder 8 steht, |
| $R^1$ | | - für Wasserstoff oder geradkettiges oder verzweigtes Alkyl mit bis zu 6 Kohlenstoffatomen steht, |
| n | | - für eine Zahl 0 oder 1 steht, |
| X | | - für Phenyl oder Pyridyl steht, die gegebenenfalls bis zu 3-fach gleich oder verschieden durch Fluor, Chlor, Brom, Cyano, Phenoxy, Trifluormethyl, Trifluormethoxy oder durch geradkettiges oder verzweigtes Alkyl mit bis zu 6 Kohlenstoffatomen substituiert sind |

und deren Salze.

Die erfindungsgemäßen Stoffe zeigen überraschenderweise eine gute thromboxansynthetasehemmende und thromboxanantagonistische Wirkung und können zur Behandlung von thromboembolischen Erkrankungen verwendet werden.

Im Rahmen der vorliegenden Erfindung werden physiologisch unbedenkliche Salze bevorzugt. Physiologisch unbedenkliche Salze der Phenylsulfonamid-substituierten Pyridinalken- und aminoalkancarbonsäurederivate können Salze der erfindungsgemäßen Stoffe mit Basen sein. Im allgemeinen seien hier Salze mit anorganischen oder organischen Basen genannt.

Salze im Rahmen der vorliegenden Erfindung sind außerdem Salze der einwertigen Metalle wie Alkalimetalle und die Ammoniumsalze. Bevorzugt werden Natrium-, Kalium-, Ammoniumsalze und Triethylammoniumsalze.

Die erfindungsgemäßen Verbindungen existieren in stereoisomeren Formen, die sich wie Bild und Spiegelbild (Enantiomere; $A=H$, $B=CH_2$, $D=CH_2$) verhalten. Die Erfindung betrifft sowohl die Antipoden als auch die Racemformen, Die Racemformen lassen sich in bekannter Weise in die stereoisomer einheitlichen Bestandteile trennen (vgl. E.L. Eliel, Stereochemistry of Carbon Compounds, McGraw Hill, 1962).

Außerdem können im Fall, daß A und B gemeinsam für die Reste =CH- oder =N- stehen und D entsprechend der oben angegebenen Bedeutung entweder für die $-CH_2$-Gruppe oder für Sauerstoff steht, isomere Formen bezüglich der Stellung der Substituenten an der Doppelbindung auftreten. Sowohl die einzelnen Isomere als auch deren Mischungen sind Gegenstand der Erfindung.

Bevorzugt sind Verbindungen der allgemeinen Formel (I),

in welcher

A - für Wasserstoff steht,

B - für die $-CH_2$-Gruppe steht,

oder

| | |
|---|---|
| A und B | gemeinsam für einen Rest der Formel =CH- oder =N- stehen, |
| D | - für die $-CH_2$-Gruppe steht oder im Fall, daß B mit A den Rest der Formel =N- bedeutet, für Sauerstoff steht, |
| m | - für eine Zahl 1, 2, 3, 4, 5 oder 6 steht, |
| $R^1$ | - für Wasserstoff oder für geradkettiges oder verzweigtes Alkyl mit bis zu 4 Kohlenstoffato- |

men steht,

n - für eine Zahl 0 oder 1 steht,

X - für Phenyl oder Pyridyl steht, die gegebenenfalls bis zu 3-fach gleich oder verschieden durch Fluor, Chlor, Cyano, Phenoxy, Trifluormethyl, Trifluormethoxy oder durch geradkettiges oder verzweigtes Alkyl mit bis zu 4 Kohlenstoffatomen substituiert sind

und deren Salze.

Außerdem wurde ein Verfahren zur Herstellung der erfindungsgemäßen Verbindungen der allgemeinen Formel (I),

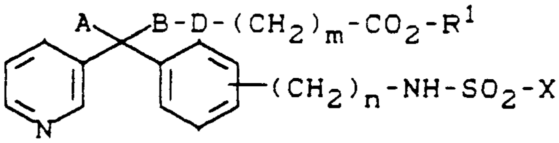

(I)

in welcher
A, B, D, m, $R^1$, n und X die oben angegebene Bedeutung haben,
gefunden, das dadurch gekennzeichnet ist, daß man

[A] im Fall, daß A und B gemeinsam für den Rest der Formel =N- stehen und D Sauerstoff bedeutet, Verbindungen der allgemeinen Formel (II),

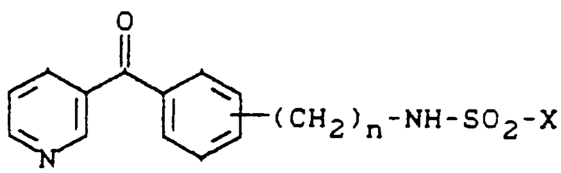

(II)

in welcher
n und X die oben angegebene Bedeutung haben,
entweder direkt mit Verbindungen der allgemeinen Formel (III)

$H_2N\text{-}O\text{-}(CH_2)_m\text{-}CO_2R^1$ (III)

in welcher
m und $R^1$ die oben angegebene Bedeutung haben,
gegebenenfalls in Form ihrer Salze, in inerten Lösemitteln, gegebenenfalls in Anwesenheit einer Base umsetzt,
oder zunächst mit Hydroxylamin nach üblicher Methode zu den entsprechenden Oximen der allgemeinen Formel (IV)

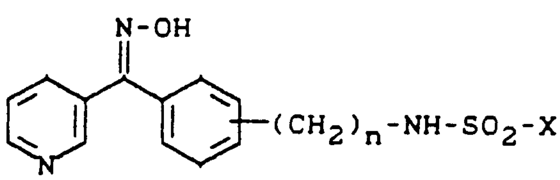

(IV)

in welcher
n und X die oben angegebene Bedeutung haben,
umsetzt
und anschließend mit Carbonsäurederivaten der allgemeinen Formel (V)

$Y\text{-}(CH_2)_m\text{-}CO_2\text{-}R^1$ (V)

in welcher
m und $R^1$ die oben angegebene Bedeutung haben
und
Y - für eine typische Abgangsgruppe steht,
in inerten Lösemitteln, in Anwesenheit einer Base umsetzt,
oder
[B] im Fall, daß A und B gemeinsam für einen Rest der Formel =CH- stehen und D die $-CH_2$-Gruppe bedeutet,
die Verbindungen der allgemeinen Formel (II) in inerten Lösemitteln, in Anwesenheit einer Base mit Ylidverbindungen der allgemeinen Formel (VI)

$(C_6H_5)_3P^{\oplus}-CH_2-(CH_2)_m-CO_2-R^1 \times Z^{\ominus}$ (VI)

in welcher
m und $R^1$ die oben angebebene Bedeutung haben
und
Z - für ein Halogenatom, vorzugsweise für Brom steht,
umsetzt
oder
[C] Verbindungen der allgemeinen Formel (VII)

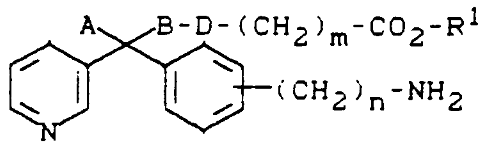
(VII)

in welcher
A, B, D, n, m und $R^1$ die oben angegebene Bedeutung haben,
mit Verbindungen dar allgemeinen Formel (VIII)

$X-SO_2-Y$ (VIII)

in welcher
X und Y die oben angegebene Bedeutung haben,
sulfoniert,
und im Fall, daß A für Wasserstoff und B und D für die $-CH_2$-Gruppe stehen, nach üblicher Methode eine katalytische Reduktion anschließt,
und in allen Verfahren [A], [B] und [C] im Fall der Säuren ($R^1$ = H) die Ester nach üblicher Methode verseift.

Die erfindungsgemäßen Verfahren können durch folgendes Formelschema beispielhaft erläutert werden:

[A]

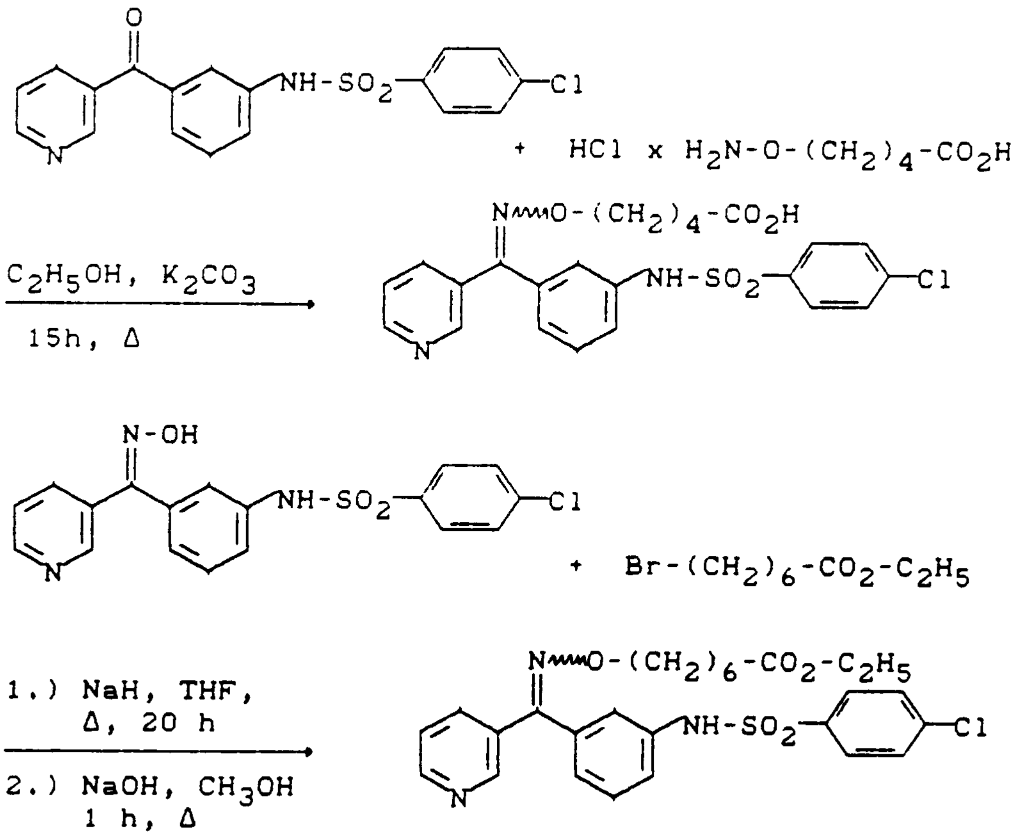

[B]

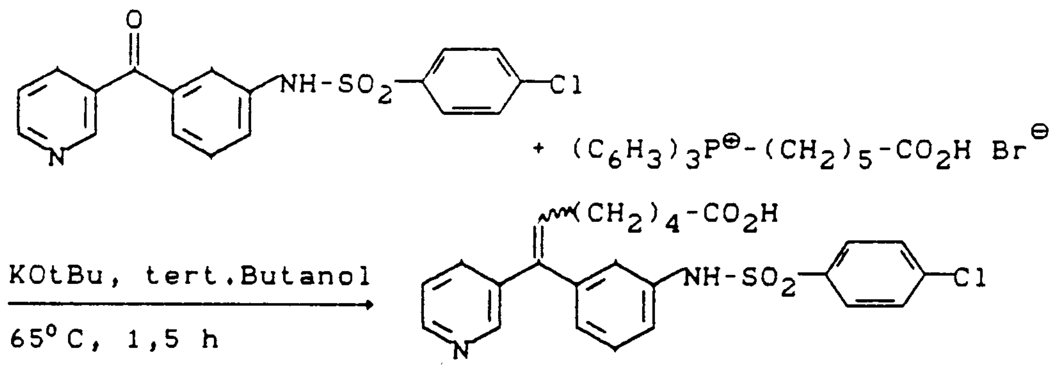


[C]

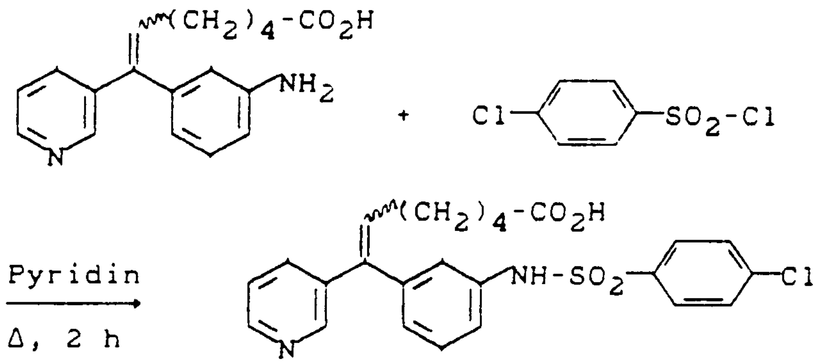


Grundsätzlich können als Lösemittel für die Verfahren [A], [B] und [C] die üblichen organischen Lösemittel, die sich unter den Reaktionsbedingungen nicht verändern, eingesetzt werden. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Besonders bevorzugt sind in Abhängigkeit der einzelnen Reaktionsschritte Ethanol, Tetrahydrofuran, Pyridin und tert.Butanol.

Als Basen bei den Verfahren [A] und [B] eignen sich beispielsweise Alkali- und Erdalkalicarbonate wie Kaliumcarbonat, Natriumcarbonat, Calciumcarbonat, Alkali- und Erdalkalihydroxide wie Natriumhydroxid, Kaliumhydroxid, Calciumhydroxid, Alkali- und Erdalkalihydride wie Natriumhydrid oder Calciumhydrid, Lithiumorganische Verbindungen wie Butyllithium oder Phenyllithium, Alkalialkoholate wie Natriummethanolat, Natriumethanolat oder Kalium-tert.butylat oder organische Basen wie beispielsweise Triethylamin oder Pyridin.

Die Umsetzungen für die Verfahren [A] und [B] können bei normalem oder erhöhtem Druck (beispielsweise 1,0 bis 5 bar), vorzugsweise bei Normaldruck durchgeführt werden.

Die Sulfonierung [C] wird im allgemeinen in einem Temperaturbereich von -80 °C bis +150 °C, bevorzugt von 0 °C bis +80 °C durchgeführt.

Die Sulfonierung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Im allgemeinen setzt man 1 bis 3 Mol, bevorzugt 1 bis 2 Mol, besonders bevorzugt 1 Mol Sulfonyl-Verbindung (VIII), bezogen auf 1 Mol des Amins (VII) ein.

Als säurebindende Mittel für die Sulfonierung können Alkali- oder Erdalkalicarbonate wie Natriumcarbonat, Kaliumcarbonat, Alkali- oder Erdalkalihydroxide wie beispielweise Natrium- oder Kaliumhydroxid, oder organische Basen wie Pyridin, Triethylamin, N-Methylpyridin, oder bicyclische Amidine wie 1,5-Diazabicyclo[3.4.0]-nonene-5 (DBN) oder 1,5-Diazabicyclo[5.4.0]undecene-5 (DBU) eingesetzt werden, Bevorzugt ist Kaliumcarbonat.

Die säurebindenden Mittel werden im allgemeinen in einer Menge von 0,5 bis 3 Mol, bevorzugt von 1 bis 1,5 Mol, bezogen auf die Verbindungen der allgemeinen Formel (VIII) eingesetzt.

Die Reduktion der Doppelbindung durch Hydrierung erfolgt im allgemeinen mit Wasserstoff in Anwesenheit eines Katalysators wie beispielsweise Platin oder Platinoxide, Rhodium, Ruthenium, Chlorotris-(triphenylphosphin)rhodium, oder Palladium auf Tierkohle, bevorzugt mit Palladium auf Tierkohle in einem Temperaturbereich von 0°C bis +150°C, bevorzugt von +25°C bis +100°C,

Als Lösemittel für die Hydrierung eignen sich protische Lösemittel wie beispielsweise Methanol, Ethanol und/oder aprotische Lösemittel wie beispielsweise Tetrahydrofuran, Toluol, Dimethylformamid, Methylenchlorid oder Dioxan.

Die Hydrierung wird bei einem Druck von 1 bis 300 atm, vorzugsweise bei 1 bis 20 atm durchgeführt.

Die eingesetzte Menge an Katalysatoren beträgt 0,5 bis 5, bevorzugt 1 bis 1,5 Molprozent bezogen auf die Verbindung der allgemeinen Formel (I, A und B = =CH-, D = $-CH_2$).

Die Verseifung der Carbonsäureester erfolgt nach üblichen Methoden, indem man die Ester in Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliumethanolat, Kaliummethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80° durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Als typische Abgangsgruppe Y in den Verbindungen der allgemeinen Formeln (V) und (VIII) eignen sich Cl, Br, J, $-OSO_2CH_3$ und $-OSO_2-C_6H_4-p-CH_3$.

Die Verbindungen der allgemeinen Formel (III) sind an sich bekannt [vgl. Tetrahedron 23, (1967), 4441-4447) oder können nach den dort beschriebenen Verfahren hergestellt werden.

Ebenso sind die Carbonsäurederivate der allgemeinen Formel (V) bekannt [vgl. Beilstein 3, 5; Fieser 1, 247; 2, 129; 3, 95; 5, 213].

Die Ylidverbindungen der allgemeinen Formel (VI) [vgl. J. Med. Chem, 28 (1985), 3, 287] und die Sulfonsäuren und ihre aktivierten Derivate der allgemeinen Formel (VIII) [vgl. Houben-Weyl's, "Methoden der organischen Chemie, Band IX, S. 407 ff.] sind bekannt oder können nach den aufgeführten Methoden hergestellt werden.

Die Verbindungen der allgemeinen Formel (II) sind neu und können hergestellt werden, indem man die oben aufgeführten Sulfonsäurederivate der allgemeinen Formel (VIII) mit Aminen der allgemeinen Formel (IX)

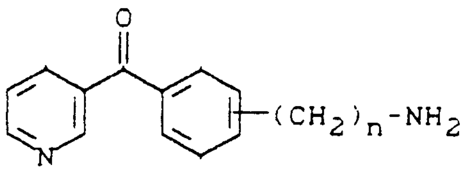

(IX)

in welcher

n die oben angegebene Bedeutung hat,

nach der unter dem Verfahren [C] angegebenen Bedingungen umsetzt.

Die Verbindungen der allgemeinen Formel (IX) sind teilweise bekannt (n = 0) [vgl. J` Med. Chem. 24 (1981) 12, 1499] oder können nach den dort beschriebenen Verfahren hergestellt werden;
im Fall, daß n die Zahl 1 bedeutet können die Verbindungen hergestellt werden, indem man in Analogie zu literaturbekannten Verfahren in den entsprechenden Hydroxy-Cyanoverbindungen (X)

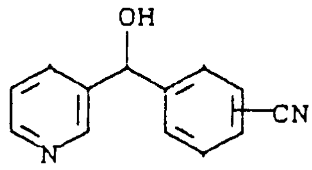 (X)

nach üblicher Methode zunächst die Hydroxy-Gruppe zur Carbonylgruppe oxidiert und anschließend die Cyano-Gruppe reduziert [vgl. dazu J. Med, Chem. 1986, 29, 1461; J. Chem. Soc. Perkin Trans 1, (1972), 1655; J. Org. Chem. 1978, 43, 4537,; Rylander "Catalytic Hydrogenation over Platinum Metals", Academ, Press., Inc., New York (1967)].

Die Verbindungen der allgemeinen Formel (X) sind bekannt [vgl. EP 221 601 A1].

Die Verbindungen der allgemeinen Formel (IV) sind ebenfalls neu und können nach der oben aufgeführten Methode hergestellt werden.

Die Verbindungen der allgemeinen Formel (VII) sind neu und können hergestellt werden, indem man Verbindungen der allgemeinen Formel (XI)

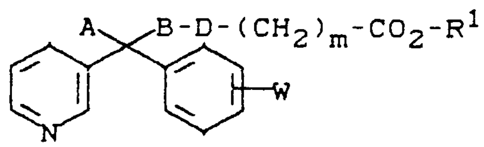 (XI)

in welcher
A, B, D, m und $R^1$ die oben aufgeführte Bedeutung haben
und

W im Fall, daß n in den Verbindungen der allgemeinen Formel (VII) für die Zahl 0 steht, Nitro, im Fall, daß n für die Zahl 1 steht, Cyano bedeutet,

nach üblicher Methode reduziert.

Die Reduktion verläuft in Analogie zu literaturbekannten Verfahren [vgl. EP 337 640; J. March "Advanced Organic Chemistry", S. 1125 (2nd Edition); Rabinowitz in Rappoport, "The Chemistry of the Cyano Group", S. 307 - 340; Interscience Publishers, New York (1970)].

Die Verbindungen der allgemeinen Formel (XI) sind bekannt [vgl. EP 221 601, 135 316].

Die erfindungsgemäßen Verbindungen, deren Salze sowie Isomere können als Wirkstoffe in Arzneimitteln eingesetzt werden. Die Stoffe weisen eine thrombozytenaggregationshemmende und Thromboxan $A_2$-antagonistische Wirkung auf und hemmen die Thromboxansyntheta an isolierten Plättchen. Sie können zur Behandlung von thromboembolischen Erkrankungen und Ischämien wie Myokardinfarkt, Hirnschlag, transitorische und ischämische Attacken, Angina pectoris, periphere Durchblutungsstörungen, Verhinderung von Restenosen wie nach Thrombolysetherapie, percutanen transluminalen Angioplastien (PTA), percutanen transluminalen Koronarangioplastien (PTCA), Bypass sowie zur Behandlung von Arteriosklerose, Asthma und Allergien eingesetzt werden.

Zur Bestimmung der thrombozytenaggregationshemmenden Wirkung wurde Blut von gesunden Probanden beiderlei Geschlechts verwendet. Als Antikoagulanz wurden einem Teil 3,8%iger wäßriger Natriumzitratlösung 9 Teile Blut zugemischt. Mittels Zentrifugation erhält man aus diesem Blut plättchenreiches Zitratplasma (PRP)[1] (Jürgens/ Beller, Klinische Methoden der Blutgerinnungsanalyse; Thieme Verlag, Stuttgart, 1959).

Für diese Untersuchungen wurden 0,8 ml (PRP)[1] und 0,1 ml der Wirkstofflösung bei 37°C im Wasserbad vorinkubiert. Anschließend wurde die Thrombozytenaggregation nach der turbidometrischen Methode (Born, G.V.R., J. Physiol. (London), 162, 67, 1962) im Aggregometer bei 37°C bestimmt (Therapeutische Berichte 47, 80-86, 1975). Hierzu wurde die vorinkubierte Probe mit 0,1 ml Kollagen, einem aggregationsauslösenden Agens, versetzt. Die Veränderung der optischen Dichte in der Probe der (PRP)

wurde während einer Zeitdauer von 6 Minuten aufgezeichnet und der Ausschlag nach 6 Minuten bestimmt. Hierzu wurde die prozentuale Hemmung gegenüber der Kontrolle errechnet.

Als Grenzkonzentration wurde der Bereich der minimal effektiven Konzentration angegeben.

| Beispiel-Nr. | TAH-Grenzkonzentration (μg/ml) |
|---|---|
| I | 0,3 - 1,0 |
| II | 1 - 3 |
| XII | 1 - 3 |
| XIII | > 10 |
| XV | 3 - 10 |
| XXVI | 0,1 - 0,3 |

Messung der Thromboxansynthetase an gewaschenen Humanthrombozyten

1. Präparation von Thrombozytensuspensionen

Blut von gesunden Spendern wird in EDTA (1% in 0,9% NaCl, 9 + 1) aufgenommen und bei 1000 U/min (150 g) 20 min zentrifugiert. Das plättchenreiche Plasma (PRP)[2] wird abgehebert und jeweils 10 ml bei 2500 U/min 20 min zentrifugiert. Das plättchenreiche Plasma[2] wird abdekantiert. Die verbleibenden Plättchen werden in 5 ml Resuspensionspuffer (0,15 M TRIS / 0,9% NaCl/ 77 mmol EDTA, 8:91:1; mit 1 N HCl auf pH 7,4 eingestellt) suspendiert, 20 min bei 2500 U/min zentrifugiert und in 1 ml Resuspensionspuffer suspendiert. Die Thrombozytenzahl wird auf 3 x $10^5$/μl eingestellt.

2. Messung der Thromboxansynthetase

1 ml der Plättchensuspension und 0,01 ml des Prüfpräparates in 10% DMSO, werden 2 min bei 37°C inkubiert. Dazu werden 0,1 ml $^3$H-Arachidonsäure Amersham Buchler GmbH und Co. KG (6,6 x $10^{-5}$ mol/l) mit einer spezifischen Aktivität von 140 MBq/mmol zugegeben und weitere 10 min bei 37°C inkubiert. Nach der Reaktion wird das Gemisch mit ca, 0,02 ml 0,5 N Citronensäure angesäuert und unmittelbar 3-mal mit je 1 ml Essigester extrahiert. Die Überstände werden in 10 ml Glasröhrchen gesammelt und der Essigester unter $N_2$ bei 25°C abgeblasen, Der Rückstand wird in 50 μl MeOH/$CHCl_3$ (1:1) aufgenommen und auf DC-Glasplatten (Kieselgel 60, F254, 20 x 20 cm, Merck) aufgetragen.

Die Trennung erfolgt in einem Fließmittelgemisch $CHCl_3$/ MeOH/Eisessig/$H_2O$ (80:8:1:0,8), Die Verteilung der Radioaktivität wird in einem DC-Scanner Ramona-Ls der Fa. Raytest erfaßt und mit einem Integrationsprogramm quantitativ ausgewertet.

Bestimmt wird die Konzentration der Prüfsubstanzen, die zu einer 50%igen Hemmung der Thromboxanbildung gegenüber der Kontrolle führt.

Hemmung der Thromboxansynthetase an gewaschenen Plättchen aus Humanblut.

| Beispiel Nr. | $IC_{50}$ mol/l |
|---|---|
| 1 | 0,5-1 x $10^{-9}$ |
| 5 | 0,5-1 x $10^{-9}$ |

Thromboxanrezeptorbindungstest an menschlichen Thrombozytenmembranen

a) Membranpräparation

Das am Vorabend nach Standardmethoden abgenommene Blut wurde morgens 10 min bei 10°C mit 2800 U/min zentrifugiert. Der dabei als Schicht zwischen dem plättchenarmen Plasma und den Erythrozyten entstandenen Buffy-Coat wurde mit 10 μM Indomethazin versetzt. Aus dem Buffy-Coat wurden nach einer Methode, die von Barber und Jamieson (vgl. Barber, A.J., IaMieson, G.A: Isolation and characterization of plasma membranes form humanyblood platelets, J. Biol. Chem. 245, 6357-6365, 1970), beschrieben wurde, Thrombozytenmembranen präpariert. Als wichtigster Schritt werden dabei Thrombozyten mit Glycerin belade und durch osmometrischen Schock zur Lyse gebracht.

Die so erhaltenen, gewaschenen Membranen wurden in Tris-NaCl-Glucose-Puffer (50 mM Tris, 100 mM NaCl, 5 mM Glucose, pH 7,4) resuspendiert, in Trockeneis schockgefroren und bei -70°C gelagert.

b) Verdrängungsstudien

Für die Verdrängungsstudien wurden 100 µg Membranprotein und ca. 5 nM $^3$H-(3R)-3-(4-Fluorphenylsulfonamido)-9-(2-carboxyethyl)-1,2,3,4-tetrahydro-4α,4β-carbazol [zur Herstellung vgl, DOS 36 31 824; die radioaktive Markierung erfolgt nach literaturbekannter Methode] in einem Gesamtvolumen von 1 ml Tris-NaCl-Glucose-Puffer inkubiert, Zum Ansatz wurden steigende Konzentrationen der verdrängenden unmarkierten erfindungsgemäßen Verbindungen gelöst in DMSO zugegeben (Endkonzentration, 0,5% DMSO, bezogen auf das Assay Volumen).

Die Substanzkonzentration $IC_{50}$, die benötigt wird, um 50% der spezifischen Bindung zu verdrängen, wurde mit Hilfe eines logit-log Plots nach HILL bestimmt. Aus der $IC_{50}$ sowie der Dissoziationskonstanten $K_D$ (bestimmt durch Scatchard-Analyse) wurde die Inhibitionskonstante $K_I$ bestimmt.

Zur vorliegenden Erfindung gehören auch pharmazeutische Zubereitungen, die neben inerten, nichttoxischen, pharmazeutisch geeigneten Hilfs- und Trägerstoffen eine oder mehrere Verbindungen der allgemeinen Formel (I) enthalten, oder die aus einem oder mehreren Wirsktoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs-oder Trägerstoffen.

Im allgemeinen hat es sich als vorteilhaft erwiesen, den oder die Wirkstoffe der Formel (I) in Gesamtmengen von etwa 0,03 bis etwa 30 mg/kg, bevorzugt bis etwa 5 mg/kg Körpergewicht je 24 Stunden, gegebenenfalls in Form mehrerer Einzelgaben, zur Erzielung des gewünschten Ergebnisses zu verabreichen.

Eine Einzelgabe enthält den oder die Wirkstoffe vorzugsweise in Mengen von 0,01 bis etwa 10, besonders bevorzugt 0,1 bis 1,0 mg/kg Körpergewicht.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament der Art und Schwere der Erkrankung, der Art der Zubereitung und Applikation, sowie dem Zeitpunkt bzw. Intervall, zu welchem die Verabreichung erfolgt.

Ausgangsverbindungen

Beispiel 1

3-(4-Chlorphenylsulfonamido)-phenyl-3-pyridinylketon

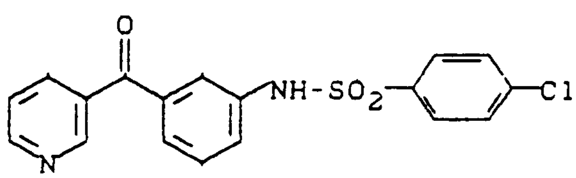

3,96 g (20 mmol) 3-Aminophenyl-3-pyridinylketon und 4,22 g (20 mmol) 4-Chlorbenzolsulfonsäurechlorid werden in 40 ml THF 24 h refluxiert. Nach Abkühlen auf Raumtemperatur wird das Lösemittel im Vakuum eingedampft und der Rückstand in 100 ml Methylenchlorid und 100 ml gesättigter Natriumhydrogencarbonatlösung aufgelöst. Die Methylenchloridphase wird abgetrennt, mit Natriumsulfat getrocknet und eingedampft. Der Rückstand wird über Kieselgel mit Methylenchlorid/Aceton 10:1 als Eluens chromatographiert.

Ausbeute: 4,98 g (66,8% der Theorie)

Fp.: 140-142 °C (nach Chromatographie, nicht umkristallisiert)

$R_f$ = 0,52 ($CH_2Cl_2$/MeOH 10:1)

Die in Tabelle 1 aufgeführten Verbindungen wurden in analoger Weise wie in Beispiel 1 beschrieben, hergestellt.

Tabelle 1

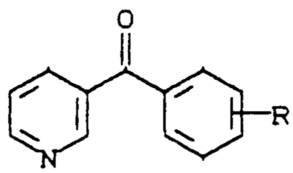

| Bsp.-Nr. | R | Fp. (nach Chromatographie nicht umkristal.) |
|---|---|---|
| 2 | 3-NH-$SO_2$ 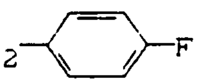 | 132° C |
| 3 | 3-NH-$SO_2$ 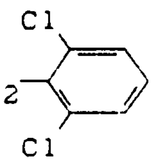 | 195° C |
| 4 | 3-NH-$SO_2$ 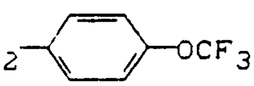 | 69° C |
| 5 | 3-NH-$SO_2$ 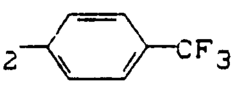 | 173° C |
| 6 | 3-NH-$SO_2$ 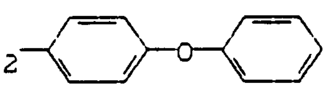 | 58° C |
| 7 | 3-NH-$SO_2$ 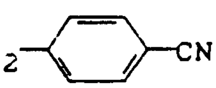 | 75° C |
| 8 | 3-NH-$SO_2$ 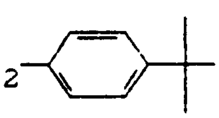 | 177° C |

Fortsetzung Tabelle 1

| Bsp.-Nr. | R | Fp. (nach Chromatographie nicht umkristal.) |
|---|---|---|
| 9 | 2-NH-$SO_2$ 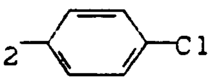 | |
| 10 | 4-NH-$SO_2$ 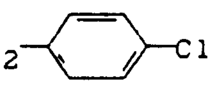 | 177-179° C |

Beispiel 11

3-(4-Chlorphenylsulfonamido)-phenyl-3-pyridinyl-ketonoxim

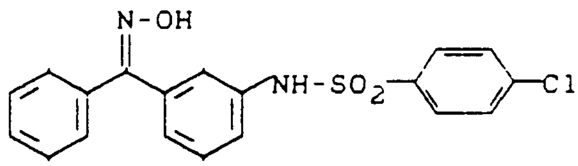

7,45 g (20 mmol) der Verbindung aus Beispiel 1, 2,08 g (30 mmol) Hydroxylammoniumchlorid und 4,14 g (30 mmol) Kaliumcarbonat werden 15 h in 20 ml Ethanol refluxiert. Nach dem Abkühlen auf Raumtemperatur wird eingedampft und der Rückstand 3 h in 50 ml Wasser verrührt (nach dieser Zeit waren keine öligen, sondern nur noch kristalline Rückstände ungelöst). Es wird abfiltriert, mit Wasser nachgewaschen und aus Methanol durch Zugabe von Wasser umkristallisiert.
Ausbeute: 5,71 g (73,7% der Theorie)
Fp.: 170 °C

Beispiel 12

3-Cyanophenyl-3-pyridinyl-methanol

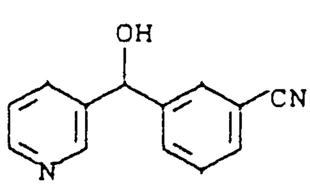

52,8 ml 2,5 molare Butyllithiumlösung (0,132 mol) in n-Hexan werden unter Argon in einem Gemisch von 66 ml abs. THF und 66 ml abs. Diethylether auf -78 °C gekühlt. Dazu werden innerhalb von 90 Min. 17,38 g (0,11 mol) 3-Brompyridin in 132 ml abs. Diethylether getropft und 10 Min. nachgerührt. Zu dieser Lösung werden 14,5 g (0,11 mol) 3-Cyanobenzaldehyd in 275 ml abs. Diethylether und 44 ml abs. THF getropft. Man läßt langsam auf Raumtemperatur kommen und rührt über Nacht bei Raumtemperatur. Danach wird in

1100 ml Eiswasser eingerührt, 2 mal mit je 550 ml Ether extrahiert und die vereinigten organischen Phasen durch Ausschütteln mit ges. NaCl-Lösung gewaschen. Nach Trocknen über $Na_2SO_4$ wird eingedampft und der Rückstand am Hochvakuum getrocknet.
Ausbeute: 15,7 g (68% der Theorie)
Fp.: 132 - 135 °C

Beispiel 13

3-Cyanophenyl-3-pyridinyl-keton

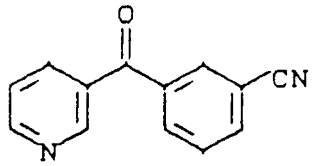

3,3 ml Oxalylchlorid werden in 75 ml abs, $CH_2Cl_2$ bei -60 °C unter Argon vorgelegt. Dazu werden zunächst 6 ml abs, DMSO in 7,5 ml abs. $CH_2Cl_2$, dann 6,3 g (30 mmol) der Verbindung aus Beispiel 12 in 75 ml abs. $CH_2Cl_2$ getropft. Man rührt 15 Min, bei -60 °C nach und tropft dann 22,5 ml Triethylamin zu, Man läßt auf Raumtemperatur kommen, rührt 3 h nach und gibt dann 30 ml Wasser zu. Das Methylenchlorid wird im Wasserstrahlvakuum abgedampft und die wäßrige Phase wird zweimal mit Essigester ausgeschüttelt. Die vereinigten Essigesterphasen werden mit Wasser gewaschen, über $Na_2SO_4$ getrocknet und eingedampft. Der Rückstand ist DC sauberes Produkt.
Ausbeute: 6,15 g (98,6% der Theorie)
$R_f$ = 0,72 ($CH_2Cl_2$/MeOH 10:1)

Beispiel 14

3-Aminomethylphenyl-3-pyridinyl-keton

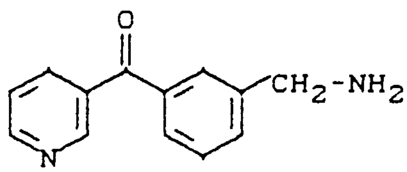

4,16 g (20 mmol) der Verbindung aus Beispiel 13 werden in 20 ml wäßriger Ammoniaklösung in Gegenwart von 1 g Ra-Ni 6 h bei 20 °C bei Normaldruck hydriert. Die wäßrige Phase wird durch Zugabe von konz. HCl sauer gestellt und 3 mal mit je 30 ml THF, dem zur besseren Phasentrennung 5 ml Essigsäureethylester beigemischt ist und extrahiert. Die vereinigten organischen Phasen werden über Natriumsulfat getrocknet und eingedampft. Der Rückstand wird über Kieselgel mit $CH_2Cl_2$/MeOH 3:1 als Eluens chromatographiert.
Ausbeute: 1,145 g (27% der Theorie)
$R_f$ = 0,46 ($CH_2Cl_2$/MeOH 3:1)

Beispiel 15

3-(4-Chlorphenylsulfonamidomethyl)phenyl-3-pyridinylketon

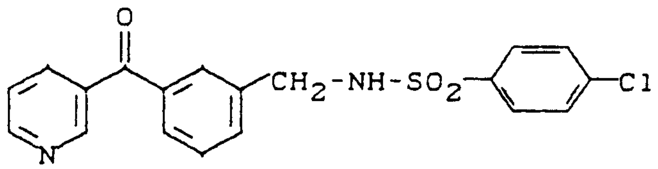

Die Verbindung aus Beispiel 14 und 4-Chlorphenylsulfonsäurechlorid werden analog Beispiel 1 umgesetzt.
Ausbeute: 54%
$R_f$ = 0,54 ($CH_2Cl_2$/MeOH 10:1)
Fp.: 78-79 °C (nach Chromatographie, nicht umkristallisiert)

Beispiel 16

(E/Z)-7-(3-Aminophenyl)-7-(3-pyridinyl)-6-hexen-carbonsäure

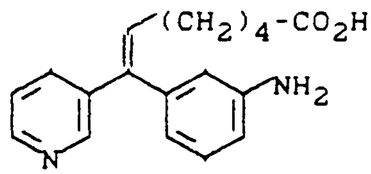

26,4 g (81 mmol) (E/Z)-7-(3-Nitrophenyl)-7-(3-pyridinyl)-6-hexen-carbonsäure [vgl. J. Med. Chem. 1985, 28, 287-294] und 52,23 g (300 mmol) $Na_2S_2O_4$ werden in 162 ml Glykolmonomethylether und 162 ml Wasser 3 h am Rückfluß gekocht. Es wird auf 60 °C abgekühlt und bei dieser Temperatur 121,5 ml konz. HCl zugetropft. Danach wird 15 Min refluxiert, abgekühlt und in 800 ml Eiswasser gegossen. Die saure Lösung wird mit gesättigter $Na_2CO_3$-Lösung auf pH 5,5 gestellt und 3 mal mit Essigester extrahiert. Nach Trocknen über $Na_2SO_4$ werden die vereinigten organischen Phasen eingedampft. Der Rückstand wird in 10%iger NaOH aufgenommen und 2 mal mit $CH_2Cl_2$ ausgeschüttelt. Die NaOH-Phase wird mit konz. HCl auf pH 5,5 eingestellt und 3 mal mit Essigester ausgeschüttelt, Nach Trocknen über $Na_2SO_4$ wird eingedampft und man erhält das saubere Produkt als E/Z-Isomere im Verhältnis von ungefähr 2:3 (E/Z bezogen auf den Pyridinring).
Ausbeute: 17.04 g (71,1% der Theorie)
$R_f$ = 0,36 ($CH_2Cl_2$/MeOH 10:1)
Die in Tabelle 2 zusammengefaßten Amine werden analog hergestellt:

Tabelle 2

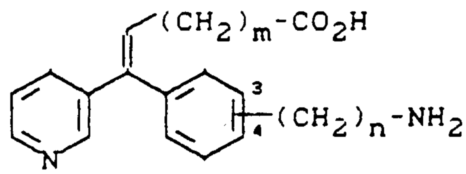

| Bsp.-Nr. | Position | m | n | $R_f$-Werte*) |
|---|---|---|---|---|
| 17 | 3 | 3 | 0 | 0,26 |
| 18 | 3 | 5 | 0 | 0,34 |
| 19 | 3 | 6 | 0 | 0,36 |
| 20 | 4 | 4 | 0 | 0,32 |
| 21 | 3 | 4 | 1 | 0,35 |

*) Laufmittelgemisch: $CH_2Cl_2$/MeOH 10:1

Herstellungsbeispiele (allgemeine Formel I)

Beispiel I

(E/Z)-5-[[[3-Pyridinyl-3-(4-chlorphenylsulfonamido)phenyl]methylen]iminooxy]pentancarbonsäure

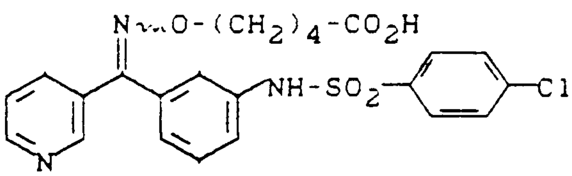

3,4 g (10 mmol) der Verbindung aus Beispiel 1 und 1,68 g (10 mmol) 5-Aminooxy-pentancarbonsäure-hydrochlorid werden zusammen mit 4,14 g (30 mmol) Kaliumcarbonat in 20 ml abs. Ethanol 15 h refluxiert. Nach dem Abkühlen auf Raumtemperatur wird in 50 ml Wasser gegossen und 2 mal mit Methylenchlorid ausgeschüttelt. Die Wasserphase wird mit 1 N HCl auf pH 5 gestellt und 3 mal mit THF, dem zur besseren Phasentrennung etwas Essigester beigegeben wird, ausgeschüttelt. Nach Trocknen über Natriumsulfat wird eingedampft und am Hochvakuum getrocknet. Die Verbindung besteht aus einem E/Z-Gemisch, enthält aber keine anderen Verunreinigungen.
Ausbeute: 3,95 g (81% der Theorie)
Fp.: 75 °C (nicht umkristallisiert)
Die in Tabelle 3 aufgeführten Verbindungen wurden in analoger Weise wie in Beispiel I beschrieben hergestellt:

Tabelle 3

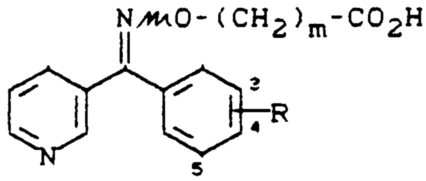

| Bsp.-Nr. | m | R | $R_f$-Wert*) |
|---|---|---|---|
| II | 4 | 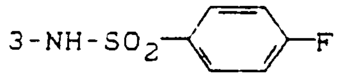 | 0,36 |
| III | 4 | 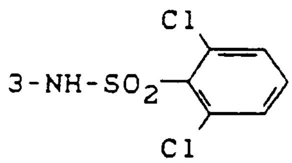 | 0,35 |
| IV | 4 | 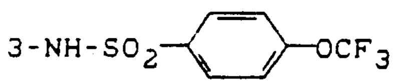 | 0,35 |
| V | 4 | 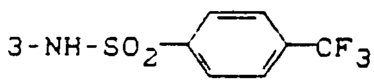 | 0,30 |
| VI | 4 | 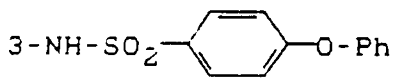 | 0,36 |
| VII | 4 | 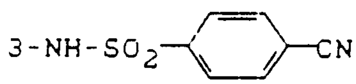 | 0,31 |
| VIII | 4 | 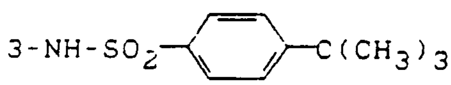 | 0,37 |
| IX | 4 | 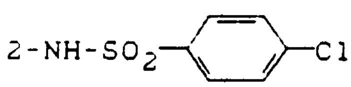 | 0,39 |
| X | 4 | 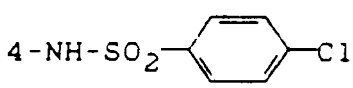 | 0,30 |
| XI | 3 | 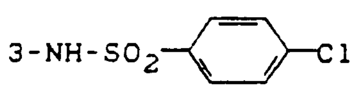 | 0,37 |
| XII | 5 | 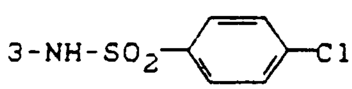 | 0,36 |

*) $CH_2Cl_2$/MeOH = 10:1

Beispiel XIII

(E/Z)-7-[[[3-Pyridinyl-3-(4-chlorphenyl-sulfonamido)phenyl]methylen]iminooxy]heptan-carbonsäure

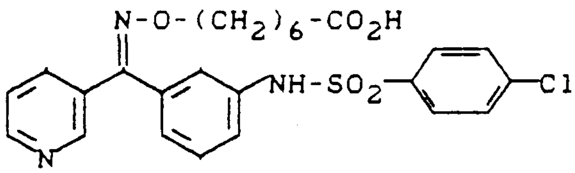

3,25 g (8,4 mmol) des Oxims aus Beispiel 11 werden in 6,3 ml abs. THF gelöst und unter Luftausschluß zu einer Suspension von 0,55 g (18,3 mmol) Natriumhydrid (80%ig in Mineralöl) in 6,3 ml abs. THF getropft. Zur besseren Rührbarkeit werden noch 20 ml abs. THF zugegeben. Es wird 1 h am Rückfluß gekocht und dann 2,19 g (9,2 mmol) 7-Bromheptancarbonsäureethylester zugetropft. Danach wird 21 h refluxiert, abfiltriert und gut mit THF nachgewaschen. Die THF-Phase wird eingedampft, der Rückstand in 30 ml Methylenchlorid aufgenommen und mit 30 ml 10%iger NaOH-Lösung und 30 ml gesättigter NaCl-Lösung gewaschen. Nach Trocknen über $Na_2SO_4$ wird eingedampft und der Rückstand über Kieselgel mit $CH_2Cl_2$/Aceton 20:1 als Eluens chromatographiert. Die das Produkt als Ethylester enthaltenden Fraktionen werden vereinigt und eingedampft. Zur Verseifung wird in 15 ml Methanol gelöst und mit 8,4 ml (10 mmol) 1 N NaOH versetzt. Es wird 3 h bei Raumtemperatur gerührt, danach 30 Min zum Rückfluß erhitzt. Das Methanol wird im Wasserstrahlvakuum abgezogen, es werden 20 ml Wasser zugegeben und mit 20 ml $CH_2Cl_2$ extrahiert. Die Wasserphase wird mit 1 N HCl auf pH 5 eingestellt und 3 mal mit je 30 ml THF, dem zur besseren Phasentrennung etwas Essigsäureethylester zugesetzt ist, extrahiert. Die THF-Phase wird über $Na_2SO_4$ getrocknet und eingedampft. Nach Trocknen am Hochvakuum erhält man das saubere Produkt.

Ausbeute: 0,51 g (11,8% der Theorie)

$R_f$ = 0,28 ($CH_2Cl_2$/MeOH 10:1)

Beispiel XIV

(E/Z)-5-[[[(3-Pyridinyl)-3-(4-chlorphenyl-sulfonamidomethyl)phenyl]methylen]iminooxy]pentancarbonsäure

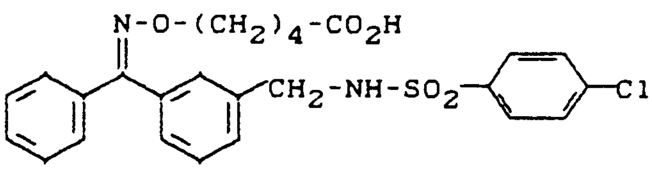

Das Keton aus Beispiel 15 wird analog Beispiel I mit 5-Aminooxy-pentancarbonsäure-hydrochlorid umgesetzt.

Ausbeute: 72%

$R_f$ = 0,36 ($CH_2Cl_2$/$CH_3OH$ 10:1)

Beispiel XV

(E/Z)-7-[3-[4-Chlorphenylsulfonamido]phenyl]-7-(3-pyridinyl)-6-heptencarbonsäure

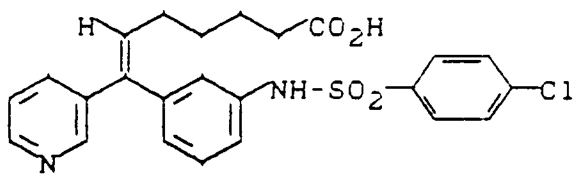

0,95 g (3,2 mmol) des Amins aus Beispiel 16 und 0,85 g (4,1 mmol) 4-Chlorbenzolsulfonsäurechlorid werden in 6,4 ml Pyridin 2 h am Rückfluß gekocht. Nach dem Abkühlen wird in 40 ml eiskalte 6 N HCl gegossen und die wäßrige Phase vom öligen Niederschlag abdekantiert. Der ölige Niederschlag wird in 40 ml Methanol gelöst und dann eingedampft. Der Rückstand wird über Kieselgel mit $CH_2Cl_2$/MeOH 10:1 als Eluens chromatographiert.
Ausbeute: 0,8 g (53,1% der Theorie)
$R_f$ = 0,7 ($CH_2Cl_2$/MeOH 10:1)
Die in Tabelle 4 zusammengefaßten Sulfonamide wurden in analoger Art und Weise hergestellt.

Tabelle 4

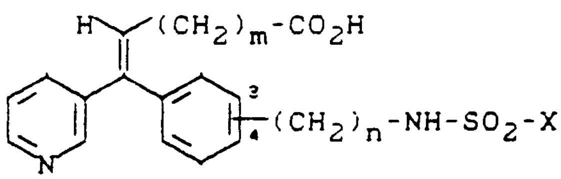

| Bsp.-Nr. | Position | m | n | X | $R_f$-Wert*) |
|---|---|---|---|---|---|
| XVI | 3 | 3 | 0 | 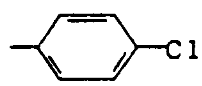 | 0,59 |

Fortsetzung Tabelle 4

| Bsp.-Nr. | Position | m | n | X | $R_f$-Wert*) |
|---|---|---|---|---|---|
| XVII | 3 | 3 | 0 | 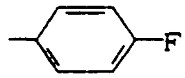 | 0,55 |
| XVIII | 3 | 4 | 0 | 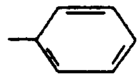 | 0,54 |
| XIX | 3 | 4 | 0 | 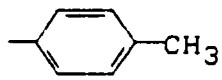 | 0,63 |
| XX | 3 | 5 | 0 | 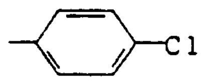 | 0,62 |
| XXI | 3 | 5 | 0 | 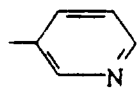 | 0,27 |
| XXII | 3 | 6 | 0 | 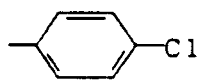 | 0,62 |
| XXIII | 3 | 6 | 0 | 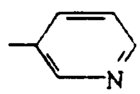 | 0,28 |
| XXIV | 4 | 4 | 0 | 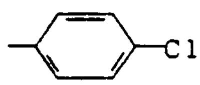 | 0,64 |
| XXV | 3 | 4 | 1 | 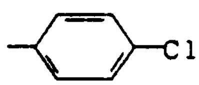 | 0,66 |

*) $CH_2Cl_2$/MeOH = 10:1

Beispiel XXVI

7-(3-[4-Chlorphenylsulfonamido]phenyl)-7-(3-pyridinyl)heptancarbonsäure

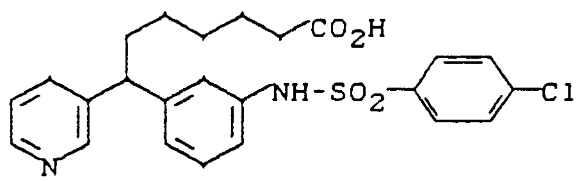

2,6 g (5,5 mmol) der Heptenarbonsäure aus Beispiel XV werden in Gegenwart von 0,51 ml (5,5 mmol) 70% Perchlorsäure in 16,5 ml abs. Ethanol mit 0,69 g Pd-C/10%ig als Katalysator bei Normaldruck und bei Raumtemperatur mit Wasserstoff hydriert. Nach 6 h war die Umsetzung im DC beendet (Ausgangssubstanz läßt sich rasch mit 0,5%iger $KMnO_4$-Lösung anfärben). Der Katalysator wird abfiltriert, es werden 30 ml gesättigter $NaHCO_3$-Lösung zugesetzt und dann 3 mal mit Essigsäureethylester extrahiert. Die vereinigten Essigesterphasen werden mit gesättigter NaCl-Lösung gewaschen, über $Na_2SO_4$ getrocknet und eingedampft. Da bei der Hydrierung der Ethylester der Heptancarbonsäure entstanden ist, wird der Rückstand direkt verseift. Dazu wird der Rückstand in 10 ml Methanol gelöst, 2,4 ml (5,8 mmol) 2 N NaOH-Lösung zugegeben und 30 Min refluxiert. Es wird eingedampft, 10 ml $H_2O$ zugegeben und 2 mal mit $CH_2Cl_2$ gewaschen. Die wäßrige Phase wird anschließend mit 10%iger HCl auf pH 5,5 gestellt und 3 mal mit Essigsäureethylester ausgeschüttelt. Die vereinigten organischen Phasen werden über $Na_2SO_4$ getrocknet und eingedampft. Der Rückstand wird über Kieselgel mit $CH_2Cl_2$/MeOH als Eluens chromatographiert.
Ausbeute: 1,38 g (53,1% der Theorie)
$R_f$ = 0,3 ($CH_2Cl_2$/MeOH 10:1)

**Patentansprüche**

**Patentansprüche für folgende Vertragsstaaten : AT, BE, CH, DE, DK, FR, GB, GR, IT, LI, LU, NL, SE**

**1.** Phenylsulfonamid-substituierte Pyridinalken- und -aminooxyalkancarbonsäurederivateder Formel I

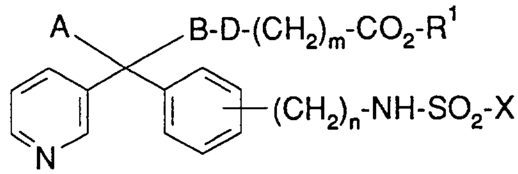

in welcher

| | |
|---|---|
| A | für Wasserstoff steht, |
| B | für die $-CH_2$-Gruppe steht, oder |
| A und B | gemeinsam für einen Rest der Formel =CH- oder =N- stehen, |
| D | für die $-CH_2$-Gruppe steht oder im Fall, daß B mit A den Rest der Formel =N- bedeutet, für Sauerstoff steht, |
| m | - für eine Zahl 1, 2, 3, 4, 5, 6, 7 oder 8 steht, |
| $R^1$ | - für Wasserstoff oder geradkettiges oder verzweigtes Alkyl mit bis zu 6 Kohlenstoffatomen steht, |
| n | - für eine Zahl 0 oder 1 steht, |
| X | - für Phenyl oder Pyridyl steht, die gegebenenfalls bis zu 3-fach gleich oder verschieden durch Fluor, Chlor, Brom, Cyano, Phenoxy, Trifluormethyl, Trifluormethoxy oder durch geradkettiges oder verzweigtes Alkyl mit bis zu 6 Kohlenstoffatomen substituiert sind |

und deren Salze.

2. Phenylsulfonamid-substituierte Pyridinalken- und -aminooxyalkancarbonsäurederivateder Formel nach Anspruch 1, in welcher

A für Wasserstoff steht,
B für $-CH_2$-Gruppe steht, oder
A und B gemeinsam für einen Rest der Formel =CH- oder =N- stehen,
D für die $-CH_2$-Gruppe steht oder im Fall, daß B mit A den Rest der Formel =N- bedeutet, für Sauerstoff steht,
m für eine Zahl 1, 2, 3, 4, 5 oder 6 steht,
$R^1$ für Wasserstoff oder für geradkettiges oder verzweigtes Alkyl mit bis zu 4 Kohlenstoffatomen steht,
n für eine Zahl 0 oder 1 steht,
X für Phenyl oder Pyridyl steht, die gegebenenfalls bis zu 3-fach gleich oder verschieden durch Fluor, Chlor, Cyano, Phenoxy, Trifluormethyl, Trifluormethoxy oder durch geradkettiges oder verzweigtes Alkyl mit bis zu 4 Kohlenstoffatomen substituiert sind

und deren Salze.

3. Phenylsulfonamid-substituierte Pyridinalken- und -aminooxyalkancarbonsäurederivatenach Anspruch 1 und 2 zur Bekämpfung von Krankheiten.

4. Verfahren zur Herstellung von Phenylsulfonamidsubstitutierten Pyridinalken- und -aminooxyalkancarbonsäurederivaten nach Anspruch 1 und 2
und deren Salze, dadurch gekennzeichnet, daß man

[A] im Fall, daß A und B gemeinsam für den Rest der Formel =N-stehen und D Sauerstoff bedeutet, Verbindungen der allgemeinen Formel (II)

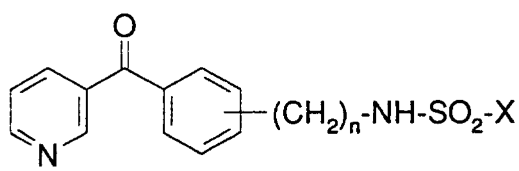

in welcher
n und X die oben angegebenen Bedeutung haben,
entweder direkt mit Verbindungen der allgemeinen Formel (III)

$H_2N-O-(CH_2)_m-CO_2-R^1$ (III)

in welcher
m und $R^1$ die oben angegebene Bedeutung haben,
gegebenenfalls in Form ihrer Salze, in inerten Lösemitteln, gegebenenfalls in Anwesenheit einer Base umsetzt,
oder zunächst mit Hydroxylamin nach üblicher Methode zu den entsprechenden Oximen der allgemeinen Formel (IV)

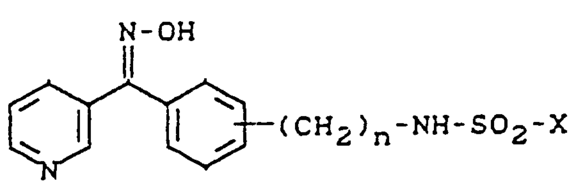
(IV)

in welcher
n und X die oben angegebene Bedeutung haben,
umsetzt
und anschließend mit Carbonsäurederivaten der allgemeinen Formel (V)

$Y-(CH_2)_m-CO_2-R^1$ (V)

in welcher
m und $R^1$ die oben angegebene Bedeutung haben
und
Y - für eine typische Abgangsgruppe steht,
in inerten Lösemitteln, in Anwesenheit einer Base umsetzt,
oder
[B] im Fall, daß A und B gemeinsam für einen Rest der Formel =CH- stehen und D die $-CH_2$-Gruppe bedeutet,
die Verbindungen der allgemeinen Formel (II) in inerten Lösemitteln, in Anwesenheit einer Base mit Ylidverbindungen der allgemeinen Formel (VI)

$(C_6H_5)_3P^{\oplus}-CH_2-(CH_2)_m-CO_2-R^1 \times Z^{\ominus}$ (VI)

in welcher
m und $R^1$ die oben angebebene Bedeutung haben
und
Z - für ein Halogenatom, vorzugsweise für Brom steht,
umsetzt
oder
[C] Verbindungen der allgemeinen Formel (VII)

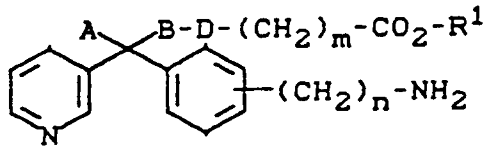
(VII)

in welcher
A, B, D, n, m und $R^1$ die oben angegebene Bedeutung haben,
mit Verbindungen der allgemeinen Formel (VIII)

$X-SO_2-Y$ (VIII)

in welcher
X und Y die oben angegebene Bedeutung haben,
sulfoniert,
und im Fall, daß A für Wasserstoff und B und D für die $-CH_2$-Gruppe stehen, nach üblicher Methode eine katalytische Reduktion anschließt,
und in allen Verfahren [A], [B] und [C[ im Fall der Säuren ($R^1$ = H) die Ester nach üblicher Methode verseift.

**5.** Arzneimittel enthalten mindestens ein Phenylsulfonamido-substituiertes Pyridinalken- oder aminooxyalkancarbonsäurederivat nach Anspruch 1 bis 2.

**6.** Verfahren zur Herstellung von Arzneimitteln nach Anspruch 5, dadurch gekennzeichnet, daß man Verbindungen der Formel (I) nach Anspruch 1 bis 2 gegebenenfalls mit Hilfe von üblichen Hilfs- und Trägerstoffen in eine geeignete Applikationsform bringt.

**7.** Verwendung von Phenylsulfonamido-substituierten Pyridinalken- und -aminooxyalkancarbonsäurederivate nach Anspruch 1 bis 2 zur Herstellung von Arzneimitteln.

**8.** Verwendung von Phenylsulfonamino-substituierten Pyridinalken- und -aminooxyalkancarbonsäurederivaten nach Anspruch 1 bis 2 zur Herstellung von Arzneimitteln zur Behandlung von thromboembolischen Erkrankungen, ischämischen Erkrankungen, Arteriosklerose, Asthma und Allergien.

**Patentanspruch für folgenden Vertragsstaat : ES**

1. Verfahren zur Herstellung von Phenylsulfonamidsubstitutierten Pyridinalken- und -aminooxyalkancarbonsäurederivaten der allgemeinen Formel (I)

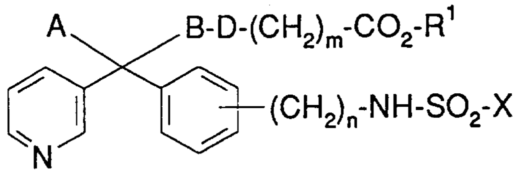

in welcher

| | |
|---|---|
| A | für Wasserstoff steht, |
| B | für die $-CH_2$-Gruppe steht, oder |
| A und B | gemeinsam für einen Rest der Formel =CH- oder =N- stehen, |
| D | für die $-CH_2$-Gruppe steht oder im Fall, daß B mit A den Rest der Formel =N- bedeutet, für Sauerstoff steht, |
| m | für eine Zahl 1, 2, 3, 4, 5, 6, 7, oder 8 steht, |
| $R^1$ | für Wasserstoff oder geradkettiges oder verzweigtes Alkyl mit bis zu 6 Kohlenstoffatomen steht, |
| n | für eine Zahl 0 oder 1 steht, |
| X | für Phenyl oder Pyrid steht, die gegebenenfalls bis zu 3-fach gleich oder verschieden durch Fluor, Chlor, Brom, Cyano, Phenoxy, Trifluormethyl, Trifluormethoxy oder durch geradkettiges oder verzweigtes Alkyl mit bis zu 6 Kohlenstoffatomen substituiert sind |

und deren Salze, dadurch gekennzeichnet, daß man

[A] im Fall, daß A und B gemeinsam für den Rest der Formel =N-stehen und D Sauerstoff bedeutet, Verbindungen der allgemeinen Formel (II)

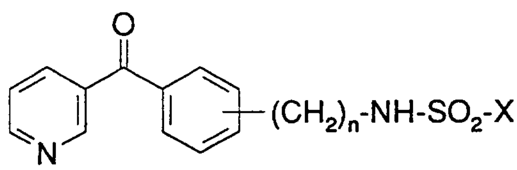

in welcher

n und X die oben angegebenen Bedeutung haben,

entweder direkt mit Verbindungen der allgemeinen Formel (III)

$H_2N-O-(CH_2)_m-CO_2-R^1$ (III)

in welcher

m und $R^1$ die oben angegebene Bedeutung haben,

gegebenenfalls in Form ihrer Salze, in inerten Losemitteln, gegebenenfalls in Anwesenheit einer Base umsetzt,

oder zunächst mit Hydroxylamin nach üblicher Methode zu den entsprechenden Oximen der allgemeinen Formel (IV)

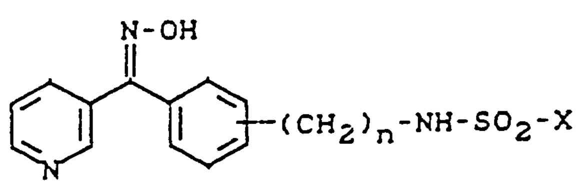

(IV)

in welcher
n und X die oben angegebene Bedeutung haben,
umsetzt
und anschließend mit Carbonsäurederivaten der allgemeinen Formel (V)

$Y\text{-}(CH_2)_m\text{-}CO_2\text{-}R^1$ (V)

in welcher
m und $R^1$ die oben angegebene Bedeutung haben
und
Y - für eine typische Abgangsgruppe steht,
in inerten Lösemitteln, in Anwesenheit einer Base umsetzt,
oder
[B] im Fall, daß A und B gemeinsam für einen Rest der Formel =CH- stehen und D die $-CH_2$-Gruppe bedeutet,
die Verbindungen der allgemeinen Formel (II) in inerten Lösemitteln, in Anwesenheit einer Base mit Ylidverbindungen der allgemeinen Formel (VI)

$(C_6H_5)_3P^{\oplus}\text{-}CH_2\text{-}(CH_2)_m\text{-}CO_2\text{-}R^1 \times Z^{\ominus}$ (VI)

in welcher
m und $R^1$ die oben angebebene Bedeutung haben
und
Z - für ein Halogenatom, vorzugsweise für Brom steht,
umsetzt
oder
[C] Verbindungen der allgemeinen Formel (VII)

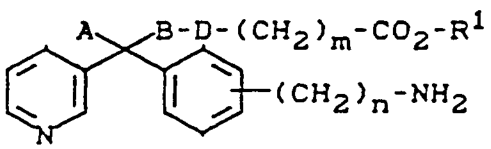
(VII)

in welcher
A, B, D, n, m und $R^1$ die oben angegebene Bedeutung haben,
mit Verbindungen der allgemeinen Formel (VIII)

$X\text{-}SO_2\text{-}Y$ (VIII)

in welcher
X und Y die oben angegebene Bedeutung haben,
sulfoniert,
und im Fall, daß A für Wasserstoff und B und D für die $-CH_2$-Gruppe stehen, nach üblicher Methode eine katalytische Reduktion anschließt,
und in allen Verfahren [A], [B] und [C] im Fall der Säuren ($R^1$ = H) die Ester nach üblicher Methode verseift.

## Claims

**Claims for the following Contracting States : AT, BE, CH, DE, DK, FR, GB, GR, IT, LI, LU, NL, SE**

1. Phenylsulphonamide-substituted pyridinealkene- and -aminooxyalkanecarboxylic acid derivatives of the formula I

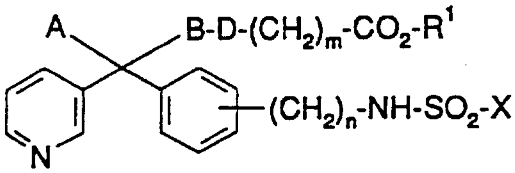

in which

| | |
|---|---|
| A | represents hydrogen, |
| B | represents the $-CH_2-$ group, or |
| A and B | together represent a radical of the formula =CH- or =N-, |
| D | represents the $-CH_2-$ group or in the case in which B with A denotes the radical of the formula =N-, represents oxygen, |
| m | - represents a number 1, 2, 3, 4, 5, 6, 7 or 8, |
| $R^1$ | - represents hydrogen or straight-chain or branched alkyl having up to 6 carbon atoms, |
| n | - represents a number 0 or 1, |
| X | - represents phenyl or pyridyl which are optionally monosubstituted to trisubstituted by identical or different substituents from the series comprising fluorine, chlorine, bromine, cyano, phenoxy, trifluoromethyl, trifluoromethoxy and straight-chain or branched alkyl having up to 6 carbon atoms |

and their salts.

**2.** Phenylsulphonamide-substituted pyridinealkene- and -aminooxyalkanecarboxylic acid derivatives of the formula according to Claim 1, in which

| | |
|---|---|
| A | represents hydrogen, |
| B | represents the $-CH_2-$ group, or |
| A and B | together represent a radical of the formula =CH- or =N-, |
| D | represents the $-CH_2-$ group or in the case in which B with A denotes the radical of the formula =N-, represents oxygen, |
| m | represents a number 1, 2, 3, 4, 5 or 6, |
| $R^1$ | represents hydrogen or straight-chain or branched alkyl having up to 4 carbon atoms, |
| n | represents a number 0 or 1, |
| X | represents phenyl or pyridyl which are optionally monosubstituted to trisubstituted by identical or different substituents from the series comprising fluorine, chlorine, cyano, phenoxy, trifluoromethyl, trifluoromethoxy and straight-chain or branched alkyl having up to 4 carbon atoms |

and their salts.

**3.** Phenylsulphonamide-substituted pyridinealkene- and -aminooxyalkanecarboxylic acid derivatives according to Claims 1 and 2 for combating diseases.

**4.** Process for the preparation of phenylsulphonamide-substituted pyridinealkene- and -aminooxyalkanecarboxylic acid derivatives according to Claims 1 and 2
and their salts, characterised in that
[A] in the case in which A and B together represent the radical of the formula =N- and D denotes oxygen,
compounds of the general formula (II)

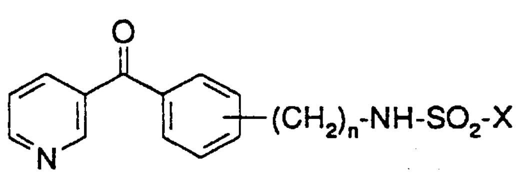

in which
n and X have the abovementioned meaning,

are either reacted directly with compounds of the general formula (III)

$H_2N-O-(CH_2)_m-CO_2-R^1$ (III)

in which
m and $R^1$ have the abovementioned meaning,
if appropriate in the form of their salts, in inert solvents, if appropriate in the presence of a base,
or are first reacted with hydroxylamine by a customary method to give the corresponding oximes of the general formula (IV)

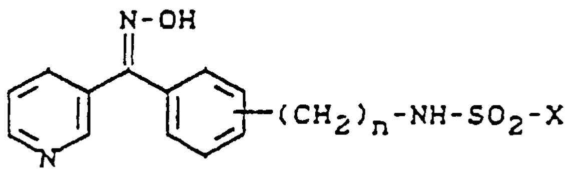

(IV)

in which
n and X have the abovementioned meaning,
and the oximes are then reacted with carboxylic acid derivatives of the general formula (V)

$Y-(CH_2)_m-CO_2-R^1$ (V)

in which
m and $R^1$ have the abovementioned meaning and
Y - represents a typical leaving group,
in inert solvents, in the presence of a base,
or
[B] in the case in which A and B together represent a radical of the formula =CH- and D denotes the $-CH_2-$ group,
the compounds of the general formula (II) are reacted in inert solvents and in the presence of a base with ylide compounds of the general formula (VI)

$(C_6H_5)_3P^{\oplus}-CH_2-(CH_2)_m-CO_2-R^1 \times Z^{\ominus}$ (VI)

in which
m and $R^1$ have the abovementioned meaning
and
Z - represents a halogen atom, preferably bromine,
or
[C] compounds of the general formula (VII)

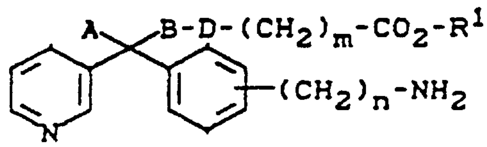

(VII)

in which
A, B, D, n, m and $R^1$ have the abovementioned meaning,
are sulphonated using compounds of the general formula (VIII)

$X-SO_2-Y$ (VIII)

in which
X and Y have the abovementioned meaning,

and in the case in which A represents hydrogen and B and D represent the $-CH_2-$ group, a catalytic reduction is added according to a customary method,
and in all processes [A], [B] and [C], in the case of the acids ($R^1$ = H), the esters are hydrolysed according to a customary method.

**5.** Medicaments containing at least one phenylsulphonamide-substituted pyridinealkene- or -aminooxyalkanecarboxylic acid derivative according to Claims 1 and 2.

**6.** Process for the production of medicaments according to Claim 5, characterised in that compounds of the formula (I) according to Claims 1 and 2 are brought into a suitable administration form, if appropriate with the aid of customary auxiliaries and excipients.

**7.** Use of phenylsulphonamide-substituted pyridinealkene- and -aminooxyalkanecarboxylic acid derivatives according to Claims 1 and 2 for the production of medicaments.

**8.** Use of phenylsulphonamide-substituted pyridinealkene- and -aminooxyalkanecarboxylic acid derivatives according to Claims 1 and 2 for the production of medicaments for the treatment of thromboembolic disorders, ischaemic disorders, arteriosclerosis, asthma and allergies.

**Claim for the following Contracting State : ES**

**1.** Process for the preparation of phenylsulphonamide-substituted pyridinealkene- and -aminooxyalkanecarboxylic acid derivatives of the general formula I

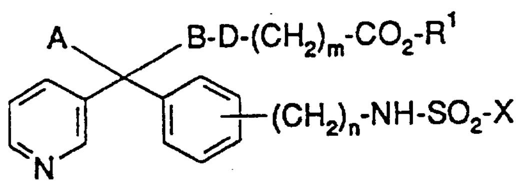

in which

| | |
|---|---|
| A | represents hydrogen, |
| B | represents the $-CH_2-$ group, or |
| A and B | together represent a radical of the formula =CH- or =N-, |
| D | represents the $-CH_2-$ group or in the case in which B with A denotes the radical of the formula =N-, represents oxygen, |
| m | represents a number 1, 2, 3, 4, 5, 6, 7 or 8, |
| $R^1$ | represents hydrogen or straight-chain or branched alkyl having up to 6 carbon atoms, |
| n | represents a number 0 or 1, |
| X | represents phenyl or pyridyl which are optionally monosubstituted to trisubstituted by identical or different substituents from the series comprising fluorine, chlorine, bromine, cyano, phenoxy, trifluoromethyl, trifluoromethoxy and straight-chain or branched alkyl having up to 6 carbon atoms |

and their salts, characterised in that

[A] in the case in which A and B together represent the radical of the formula =N- and D denotes oxygen,
compounds of the general formula (II)

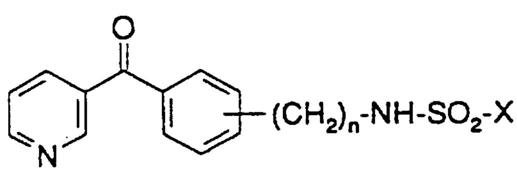

in which

n and X have the abovementioned meaning,
are either reacted directly with compounds of the general formula (III)

$H_2N$-O-$(CH_2)_m$-$CO_2$-$R^1$ (III)

in which
m and $R^1$ have the abovementioned meaning,
if appropriate in the form of their salts, in inert solvents, if appropriate in the presence of a base,
or are first reacted with hydroxylamine by a customary method to give the corresponding oximes of the general formula (IV)

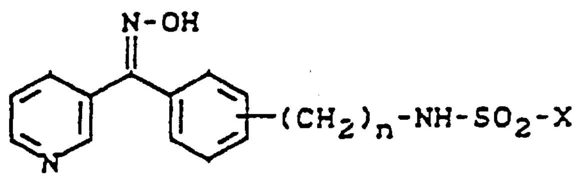
(IV)

in which
n and X have the abovementioned meaning,
and the oximes are then reacted with carboxylic acid derivatives of the general formula (V)

Y-$(CH_2)_m$-$CO_2$-$R^1$ (V)

in which
m and $R^1$ have the abovementioned meaning and
Y - represents a typical leaving group,
in inert solvents, in the presence of a base,
or
[B] in the case in which A and B together represent a radical of the formula =CH- and D denotes the -$CH_2$- group,
the compounds of the general formula (II) are reacted in inert solvents and in the presence of a base with ylide compounds of the general formula (VI)

$(C_6H_5)_3P^{\oplus}$-$CH_2$-$(CH_2)_m$-$CO_2$-$R^1$ x $Z^{\ominus}$ (VI)

in which
m and $R^1$ have the abovementioned meaning
and
Z - represents a halogen atom, preferably bromine,
or
[C] compounds of the general formula (VII)

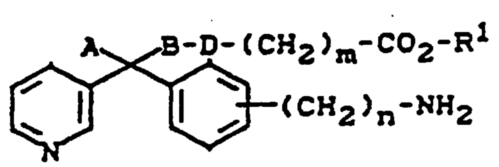
(VII)

in which
A, B, D, n, m and $R^1$ have the abovementioned meaning,
are sulphonated using compounds of the general formula (VIII)

X-$SO_2$-Y (VIII)

in which

X and Y have the abovementioned meaning,
and in the case in which A represents hydrogen and B and D represent the $-CH_2-$ group, a catalytic reduction is added according to a customary method,
and in all processes [A], [B] and [C], in the case of the acids ($R^1$ = H), the esters are hydrolysed according to a customary method.

**Revendications**

**Revendications pour les Etats contractants suivants : AT, BE, CH, DE, DK, FR, GB, GR, IT, LI, LU, NL, SE**

1. Dérivés à substituant phénylsulfonamide d'acides pyridinalcène-carboxyliques et d'acides pyridineamino-oxyalcane-carboxyliques de formule I

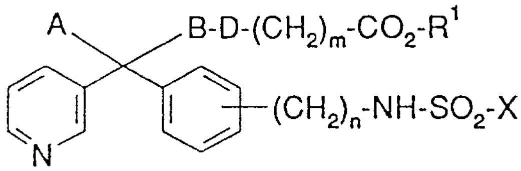

dans laquelle

| | |
|---|---|
| A | représente de l'hydrogène, |
| B | est le groupe $-CH_2-$, ou bien |
| A et B | forment conjointement un reste de formule =CH- ou =N-, |
| D | est le groupe $-CH_2-$, ou bien représente de l'oxygène au cas où B forme conjointement avec A le reste de formule =N-, |
| m | est un nombre égal à 1, 2, 3, 4, 5, 6, 7 ou 8, |
| $R^1$ | représente de l'hydrogène ou un groupe alkyle linéaire ou ramifié ayant jusqu'à 6 atomes de carbone, |
| n | est le nombre 0 ou 1, |
| X | est un groupe phényle ou un groupe pyridyle qui porte chacun le cas échéant jusqu'à 3 substituants, identiques ou différents, fluoro, chloro, bromo, cyano, phénoxy, trifluorométhyle, trifluorométhoxy ou bien alkyle linéaire ou ramifié ayant jusqu'à 6 atomes de carbone, |

et leurs sels.

2. Dérivés à substituant phénylsulfonamide d'acides pyridinalcène-carboxyliques et pyridine-aminooxyalcane-carboxyliques de formule suivant la revendication 1, dans laquelle

| | |
|---|---|
| A | représente de l'hydrogène, |
| B | est le groupe $-CH_2-$, ou bien |
| A et B | forment conjointement un reste de formule =CH- ou =N-, |
| D | représente le groupe $-CH_2-$, ou de l'oxygène au cas où B forme avec A le reste de formule =N-, |
| m | est un nombre égal à 1, 2, 3, 4, 5 ou 6, |
| $R^1$ | est de l'hydrogène ou un groupe alkyle linéaire ou ramifié ayant jusqu'à 4 atomes de carbone, |
| n | représente le nombre 0 ou 1, |
| X | est un groupe phényle ou un groupe pyridyle qui portent le cas échéant jusqu'à 3 substituants, identiques ou différents, fluoro, chloro, cyano, phénoxy, trifluorométhyle, trifluorométhoxy, ou bien alkyle linéaire ou ramifié ayant jusqu'à 4 atomes de carbone, |

et leurs sels.

3. Dérivés à substituant phénylsulfonamide d'acides pyridinalcène-carboxyliques et pyridine-aminooxyalcane-carboxyliques suivant les revendications 1 et 2, destinés à combattre des maladies.

4. Procédé de production de dérivés à substituant phénylsulfonamide d'acides pyridinalcène-carboxyliques et pyridine-amino-oxyalcane-carboxyliques suivant les revendications 1 et 2

et de leurs sels, caractérisé en ce que

[A] au cas où A et B forment ensemble le reste de formule =N- et D est de l'oxygène, on fait réagir des composés de formule générale (II)

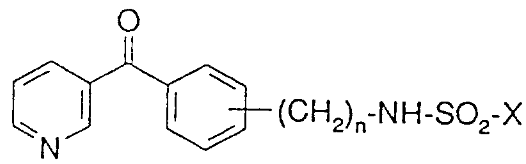

dans laquelle

n et X ont la définition indiquée ci-dessus,

soit directement avec des composés de formule générale (III)

$H_2N-O-(CH_2)_m-CO_2-R^1$ (III)

dans laquelle

m et $R^1$ ont la définition indiquée ci-dessus,

le cas échéant sous la forme de leurs sels, dans des solvants inertes, éventuellement en présence d'une base,

ou bien on les fait réagir tout d'abord avec l'hydroxylamine selon un procédé classique pour former les oximes correspondantes de formule générale (IV)

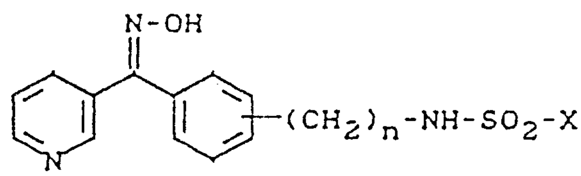 (IV)

dans laquelle

n et X ont la définition indiquée ci-dessus,

que l'on fait réagir ensuite avec des dérivés d'acides carboxyliques de formule générale (V)

$Y-(CH_2)_m-CO_2-R^1$ (V)

dans laquelle

m et $R^1$ ont la définition indiquée ci-dessus

et

Y est un groupe partant typique,

dans des solvants inertes, en présence d'une base,

ou bien

[B] au cas où A et B forment ensemble un reste de formule =CH- et D désigne le groupe $-CH_2-$, on fait réagir les composés de formule générale (II) dans des solvants inertes, en présence d'une base, avec des composés de type ylide de formule générale (VI)

$(C_6H_5)_3P^{\oplus}-CH_2-(CH_2)_m-CO_2-R^1 \times Z^{\ominus}$ (VI)

dans laquelle

m et $R^1$ ont la définition indiquée ci-dessus

et

Z représente un atome d'halogène, de préférence un atome de brome,

ou bien

[C] on sulfone des composés de formule générale (VII)

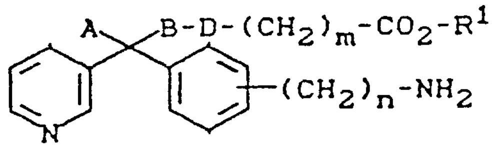

(VII)

dans laquelle
A, B, D, n, m et $R^1$ ont la définition indiquée ci-dessus, avec des composés de formule générale (VIII)

$X-SO_2-Y$ (VIII)

dans laquelle
X et Y ont la définition indiquée ci-dessus,
et au cas où A représente de l'hydrogène et B et D représentent le groupe $-CH_2-$, on effectue ensuite une réduction catalytique selon un mode opératoire classique,
et dans tous les procédés [A], [B] et [C], dans le cas des acides ($R^1$ = H), on saponifie les esters par un mode opératoire classique.

**5.** Médicaments contenant au moins un dérivé à substituant phénylsulfonamide d'acide pyridinalcènecarboxylique ou d'acide pyridine-amino-oxyalcane-carboxylique suivant les revendications 1 et 2.

**6.** Procédé de préparation de médicaments suivant la revendication 5, caractérisé en ce qu'on fait passer sous une forme convenant pour l'administration, des composés de formule (I) suivant les revendications 1 et 2, le cas échéant à l'aide de substances auxiliaires et de supports classiques.

**7.** Utilisation de dérivés à substituant phénylsulfonamide d'acides pyridinalcène-carboxyliques et pyridine-amino-oxyalcane-carboxyliques suivant les revendications 1 et 2 pour la préparation de médicaments.

**8.** Utilisation de dérivés à substituant phénylsulfonamide d'acides pyridinalcène-carboxyliques et pyridine-amino-oxyalcane-carboxyliques suivant les revendications 1 et 2 pour la préparation de médicaments destinés au traitement de maladies thromboemboliques, de maladies ischémiques, de l'artériosclérose, de l'asthme et d'allergies.

**Revendication pour l'Etat contractant suivant : ES**

**1.** Procéde de production de dérivés à substituant phénylsulfonamide d'acides pyridinalcène-carboxyliques et pyridine-amino-oxyalcane-carboxyliques de formule générale (I)

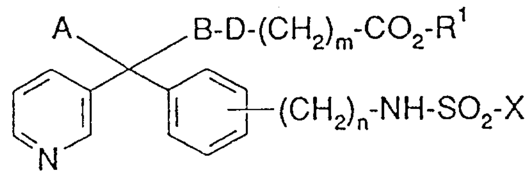

dans laquelle

| | |
|---|---|
| A | représente de l'hydrogène, |
| B | est le groupe $-CH_2-$, ou bien |
| A et B | forment conjointement un reste de formule =CH- ou =N-, |
| D | est le groupe $-CH_2-$, ou bien représente de l'oxygène au cas où B forme conjointement avec A le reste de formule =N-, |
| m | est un nombre égal à 1, 2, 3, 4, 5, 6, 7 ou 8, |
| $R^1$ | représente de l'hydrogène ou un groupe alkyle linéaire ou ramifié ayant jusqu'à 6 atomes |

de carbone,

n est le nombre 0 ou 1,

X est un groupe phényle ou un groupe pyridyle qui porte chacun le cas échéant jusqu'à 3 substituants, identiques ou différents, fluoro, chloro, bromo, cyano, phénoxy, trifluorométhyle, trifluorométhoxy ou bien alkyle linéaire ou ramifié ayant jusqu'à 6 atomes de carbone,

et de leurs sels, caractérisé en ce que

[A] au cas où A et B forment ensemble le reste de formule =N- et D est de l'oxygène, on fait réagir des composés de formule générale (II)

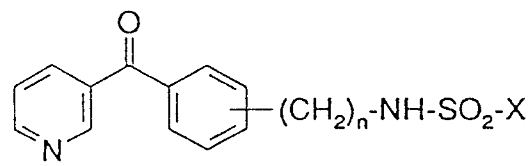

dans laquelle

n et X ont la définition indiquée ci-dessus,

soit directement avec des composés de formule générale (III)

$H_2N\text{-}O\text{-}(CH_2)_m\text{-}CO_2\text{-}R^1$ (III)

dans laquelle

m et $R^1$ ont la définition indiquée ci-dessus,

le cas échéant sous la forme de leurs sels, dans des solvants inertes, éventuellement en présence d'une base,

ou bien on les fait réagir tout d'abord avec l'hydroxylamine selon un procédé classique pour former les oximes correspondantes de formule générale (IV)

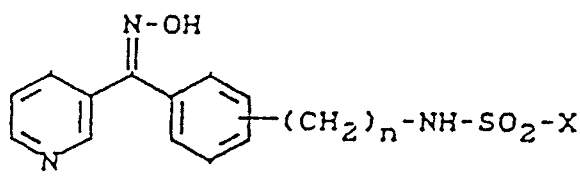 (IV)

dans laquelle

n et X ont la définition indiquée ci-dessus,

que l'on fait réagir ensuite avec des dérivés d'acides carboxyliques de formule générale (V)

$Y\text{-}(CH_2)_m\text{-}CO_2\text{-}R^1$ (V)

dans laquelle

m et $R^1$ ont la définition indiquée ci-dessus

et

Y est un groupe partant typique,

dans des solvants inertes, en présence d'une base, ou bien

[B] au cas où A et B forment ensemble un reste de formule =CH- et D désigne le groupe $-CH_2-$, on fait réagir les composés de formule générale (II) dans des solvants inertes, en présence d'une base, avec des composés de type ylique de formule générale (VI)

$(C_6H_5)_3P^{\oplus}\text{-}CH_2\text{-}(CH_2)_m\text{-}CO_2\text{-}R^1 \times Z^{\ominus}$ (VI)

dans laquelle

m et $R^1$ ont la définition indiquée ci-dessus

et

Z représente un atome d'halogéne, de préférence un atome de brome,

ou bien

[C] on sulfone des composés de formule générale (VII)

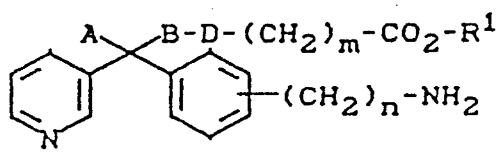

(VII)

dans laquelle

A, B, D, n, m et $R^1$ ont la définition indiquée ci-dessus, avec des composés de formule générale (VIII)

$X-SO_2-Y$ (VIII)

dans laquelle

X et Y ont la définition indiquée ci-dessus,

et au cas où A représente de l'hydrogène et B et D représentent le groupe $-CH_2-$, on effectue ensuite une réduction catalytique selon un mode opératoire classique,

et dans tous les procédés [A], [B] et [C], dans le cas des acides ($R^1$ = H), on saponifie les esters par un mode opératoire classique.